# FASCICULE DE BREVET EUROPEEN

(11) **EP 2 475 351 B1**
(45) Date de publication et mention de la délivrance du brevet: **25.02.2015**
(21) Numéro de dépôt: 10763792.8
(22) Date de dépôt: 01.09.2010
(51) Int. Cl.: A61K 8/81, A61K 47/32, A61Q 5/00, A61Q 19/00, C08F 220/28, C08F 220/56, C08F 220/58

(54) **NOUVEAU POLYMERE EN POUDRE, PROCEDE POUR SA PREPARATION ET UTILISATION COMME EPAISSISSANT**
NEUES PULVERFÖMIGES POLYMER, HERSTELLUNGSVERFAHREN DAFÜR UND SEINE VERWENDUNG ALS VERDICKER
NOVEL POWDERED POLYMER, PREPARATION METHOD THEREOF AND USE OF SAME AS A THICKENER

(30) Priorité: 11.09.2009 FR 0956224
(43) Date de publication de la demande: 18.07.2012
(73) Titulaire: Societe D'Exploitation De Produits Pour Les Industries Chimiques Seppic, 75007 Paris (FR)
(72) Inventeur: BRAUN, Olivier, F-81100 Castres (FR); MALLO, Paul, F-78290 Croissy- Sur- Seine (FR)
(74) Mandataire: Conan, Philippe Claude
(86) Numéro de dépôt international: PCT/FR2010/051819
(87) Numéro de publication internationale: WO 2011/030044

(56) Documents cités:
- EP-A1- 1 426 436
- WO-A1-2006/002936

## Description

L'invention a pour objet de nouveaux agents épaississants ainsi que leur utilisation en cosmétique et en pharmacie.

L'industrie cosmétique et l'industrie pharmaceutique utilisent très régulièrement des polymères épaississants synthétiques pour augmenter la viscosité des crèmes, des émulsions et de diverses solutions topiques. Les polymères épaississants synthétiques actuellement utilisés en ces domaines, se présentent sous deux formes physiques, la forme poudre et la forme liquide pour laquelle le polymère est préparé par polymérisation en émulsion inverse à l'aide d'agents tensioactifs et que l'on appelle couramment latex inverse.

Les polymères épaississants sous forme de poudre, les plus connus sont les polymères à base d'acide acrylique ou les copolymères à base d'acide acrylique et de ses esters. On citera par exemple, les polymères commercialisés par la société Noveon sous le nom de marque, CARBOPOL™ et PEMULEN™. Ils sont décrits notamment dans les brevets américains US 5, 373,044, US 2,798,053 et dans le brevet européen EP 0 301 532. En cosmétique, on utilise aussi et toujours sous forme de poudre, des homopolymères ou copolymères à base d'acide 2-acrylamido-2-méthyl-propane sulfonique. Ces polymères épaississants sont commercialisés sous le nom de marque Aristoflex™ et décrits notamment dans les brevets européens EP 816 403, EP 1 116 733 et EP 1 069142. Ces épaississants sous forme de poudre sont obtenus par polymérisation précipitante ; le (ou les) monomère(s) est (ou sont) mis en solution dans un solvant organique type benzène, acétate d'éthyle, cyclohexane, tertio-butanol. L'industrie cosmétique utilise aussi très largement des épaississants sous forme de latex inverses et notamment ceux commercialisés par la demanderesse. On citera par exemple, les épaississants Sepigel™ 305, Simulgel™ 600, Simulgel™ EG, Simulgel™ EPG, Simulgel™ NS, Simulgel™ A, Sepiplus™ 400, Sepiplus™ 250 et Sepiplus™ 265. Ces épaississants sont obtenus par polymérisation en émulsion inverse. Ils présentent l'avantage d'être plus aisément manipulables et se dispersent très rapidement dans l'eau. De plus, ces produits développent des performances épaississantes remarquablement élevées ; ces performances sont probablement la conséquence du procédé pour leur préparation, une réaction de polymérisation en phase dispersée, qui conduit à des polymères de très hauts poids moléculaires.

La demande internationale publiée sous le numéro WO 2006/002936 divulgue des copolymères hydrosolubles qui sont utilisés comme agents rétenteurs d'eau, stabilisants et modificateurs de rhéologie pour les ciments, plâtres, mortiers, peintures à l'eau et autres compositions fondées sur des liants aqueux. Ces copolymères sont obtenus à partir de monomères fonction acide sulfonique, de monomères à fonction amine et de monomères dérivés de (méth)acrylate d'alkyle polyéthoxylés. Cette demande internationale divulgue plus particulièrement, un terpolymère de l'acide 2-méthyl 2-[(1-oxo 2-propènyl) amino] 1-propanesulfonique partiellement salifié ou totalement salifié, de l'acrylamide et du méthacrylate de stéaryle pentacosa-éthoxylé (monomère commercialisé sous le nom PLEX 6877-O™.

Néanmoins, ces épaississants sous forme de latex inverse contiennent une huile et un ou plusieurs agents tensioactifs qui peuvent parfois induire des réactions d'intolérance cutanée sur des sujets particulièrement sensibles ; de plus cette présence d'huile les rend inutilisables pour la préparation de gels clairs.

Les inventeurs ont donc développé des systèmes épaississants comme ceux décrits dans la demande brevet français publiée sous le numéro 2 910 899, qui divulgue un terpolymère linéaire, branché ou réticulé d'au moins un monomère possédant une fonction acide fort, libre, partiellement salifiée ou totalement salifiée, avec au moins un monomère neutre, et au moins un monomère de formule (A) : 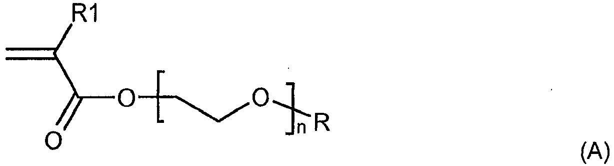
(A) dans laquelle R1 représente un atome d'hydrogène ou un radical méthyle, R représente un radical alkyle linéaire ou ramifié comportant de huit à trente atomes de carbone et n représente un nombre supérieur ou égal à un et inférieur ou égal à cinquante. Ces polymères présentent des propriétés épaississantes très prononcées, notamment en présence d'électrolytes. Ils fonctionnent sur une large gamme de pH et permettent de réaliser des gels transparents. Cependant, les formulations à bas pH épaissies par certains d'entre eux n'ont pas une tenue aux sels satisfaisante sur le long terme et certaines d'entres elles, qui contiennent des alcools gras, ont un aspect élastique peu engageant et donnant des sensations de collant au toucher et/ou un aspect d'une crème ou d'une émulsion granuleuse et non continues.

La demanderesse a mis en évidence que ces inconvénients pouvaient être évités en sélectionnant certains de ces terpolymères qui n'avaient pas été divulgués dans la demande de brevet français publiée sous le numéro 2 910 899.

C'est pourquoi selon un premier aspect l'invention a pour objet un polyélectrolyte anionique réticulé, issu de la polymérisation de l'acide 2-méthyl 2-[(1-oxo 2-propènyl) amino] 1-propanesulfonique partiellement salifié ou totalement salifié, avec le N,N-diméthyl acrylamide, et au moins un monomère de formule (I) : 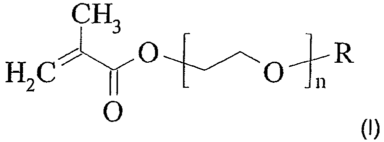 dans laquelle R représente un radical alkyle linéaire ou ramifié comportant de huit à vingt atomes de carbone et n représente un nombre supérieur ou égal à un et inférieur ou égal à vingt choisi parmi le méthacrylate de lauryle tétraéthoxylé ou le méthacrylate de stéaryle eicosaéthoxylé, en présence d'au moins un agent de réticulation.

Par polymère branché, on désigne un polymère non linéaire qui possède des chaînes pendantes de manière à obtenir, lorsque ce polymère est mis en solution dans l'eau, un fort état d'enchevêtrement conduisant à des viscosités, à bas gradient de vitesse, très importantes. Par polymère réticulé, on désigne un polymère non linéaire se présentant à l'état de réseau tridimensionnel insoluble dans l'eau, mais gonflable à l'eau et conduisant donc à l'obtention d'un gel chimique. La composition selon l'invention peut comporter des motifs réticulés et/ou des motifs branchés.

Selon un autre mode particulier, le polyélectrolyte anionique est réticulé et/ou branché avec un composé diéthylénique ou polyéthylènique dans la proportion molaire exprimée par rapport aux monomères mis en oeuvre, de 0,005% à 1%, plus particulièrement de 0,01% à 0,5% et tout particulièrement de 0,01% à 0,25%.

L'agent de réticulation et/ou l'agent de ramification est plus particulièrement choisi parmi le diméthacrylate d'éthylèneglycol, le tétraallyloxyéthane, le diacrylate d'éthylèneglycol, le diallyl urée, le triallyl amine, le triméthylol propanetriacrylate ou le méthylène-bis(acrylamide) ou un mélange de ses composés.

Dans le cadre de la présente invention, le polyélectrolyte anionique tel que défini précédemment, comporte généralement entre 5% molaire et 95% molaire de monomères à fonction acide fort, plus particulièrement entre 10% molaire et 90% molaire et tout particulièrement entre 20% molaire et 80% molaire.

Dans le cadre de la présente invention, la fonction acide fort est partiellement ou totalement salifiée comme sel de métal alcalin tel que par exemple le sel de sodium ou le sel de potassium ou comme sel d'ammonium.

Selon un autre aspect particulier, l'invention a pour objet un polyélectrolyte anionique tel que défini précédemment, dans lequel le monomère à fonction acide fort est l'acide 2-méthyl 2-[(1-oxo 2-propènyl) amino] 1-propanesulfonique partiellement ou totalement salifié sous forme de sel d'ammonium.

Dans le cadre de la présente invention le polyélectrolyte anionique tel que défini précédemment, comporte plus particulièrement entre 4,9% molaire et 90% molaire de monomère neutre, plus particulièrement entre 9,5% molaire et 85% molaire et tout particulièrement entre 15% molaire et 75% molaire.

Lorsque le monomère de formule (I) est le méthacrylate de lauryle tétraéthoxylé, il correspond au composé de formule (I) dans laquelle le radical R représente le radical dodécyle et n est égal à 4.

Lorsque que le monomère de formule (I) est le méthacrylate de stéaryle eicosaéthoxylé, il correspond au composé de formule (I) dans laquelle le radical R représente le radical octadécyle et n est égal à 20.

Dans le cadre de la présente invention le polyélectrolyte anionique tel que défini précédemment, comporte entre 0,1% molaire et 10% molaire de monomères de formule (I) et plus particulièrement entre 0,5% molaire et 5% molaire.

Le polyélectrolyte objet de la présente invention, peut également comprendre divers additifs, tels que des agents complexants, des agents de transfert ou des agents limiteurs de chaîne.

L'invention a plus particulièrement pour objet un polyélectrolyte anionique choisi parmi les polymères suivants :
- Terpolymère de l'acide 2-méthyl 2-[(1-oxo 2-propènyl)amino]1-propanesulfonique totalement salifié sous forme de d'ammonium, du N,N-diméthyl acrylamide et du méthacrylate de lauryle tétraéthoxylé, réticulé au triméthylol propanetriacrylate ;
- Terpolymère de l'acide 2-méthyl 2-[(1-oxo 2-propènyl) amino] 1-propanesulfonique totalement salifié sous forme de sel d'ammonium, du N,N-diméthyl acrylamide et du méthacrylate de stéaryle d'eicosaéthoxylé, réticulé au triméthylol propanetriacrylate.

Selon un autre aspect particulier, l'invention a pour objet un polyélectrolyte anionique réticulé, tel que défini précédemment, comportant pour 100% de monomères mis en oeuvre :
- De 20% molaire à 80% molaire d'unités monomériques issues d'un monomère comportant une fonction acide fort ;
- De 15% molaire à 75% molaire d'unités monomériques issues d'un monomère neutre ;
- De 0,5% à 5% molaire d'unités monomériques issues d'un monomère de formule (I) telle que définie précédemment.

Selon un autre aspect plus particulier, l'invention a pour objet un polyélectrolyte anionique réticulé, tel que défini précédemment, comportant pour 100% de monomères mis en oeuvre :
- De 55% molaire à 80% molaire d'unités monomériques issues d'un monomère comportant une fonction acide fort ;
- De 15% molaire à 40% molaire d'unités monomériques issues d'un monomère neutre ;
- De 1% à 5% molaire d'unités monomériques issues d'un monomère de formule (I) telle que définie précédemment.

L'invention a aussi pour objet un procédé de préparation du polélectrolyte tel que défini précédemment, caractérisé en ce que :
a) - on mélange l'ensemble des monomères, l'agent réticulant et/ ou les autres additifs dans un solvant organique, de préférence le tert-butanol,
b) - l'on amorce la réaction de polymérisation par introduction dans la dispersion préparée à l'étape a), d'un initiateur de radicaux libres, puis on la laisse se dérouler,
c) lorsque la polymérisation est terminée, on recueille le précipité par évaporation du solvant ou filtration, puis
d) on sèche le précipité.

Selon une mise en oeuvre préférée du procédé, tel que défini précédemment, la réaction de polymérisation est amorcée à une température égale ou supérieure à 50°C à l'aide d'un amorceur radicalaire produisant des radicaux par homolyse, tel que le peroxyde de dilauroyle, l'azo-bis-isobutyronitrile ou encore les dérivés azoïques.

Selon une autre mise en oeuvre du procédé, tel que défini précédemment, la réaction de polymérisation est amorcée par un couple oxydo-réducteur, à une température inférieure ou égale à 20°C.

L'invention a aussi pour objet l'utilisation du polyélectrolyte anionique tel que défini précédemment, comme épaississant et/ou comme stabilisant et/ou comme émulsionnant, d'une composition topique cosmétique, dermopharmaceutique ou pharmaceutique.

Une composition topique selon l'invention, destinée à être appliquée sur la peau ou les muqueuses de l'homme ou de l'animal, peut consister en une émulsion topique comprenant au moins une phase aqueuse et au moins une phase huile. Cette émulsion topique peut être du type huile-dans-eau (H/E), eau-dans-huile (E/H), huile-dans-eau-dans-huile (H/E/H) ou eau-dans-huile-dans-eau (E/H/E). La phase huile de l'émulsion topique peut consister en un mélange d'une ou plusieurs huiles.

Une composition topique selon l'invention peut être destinée à une utilisation cosmétique ou être utilisée pour préparer un médicament destiné au traitement des maladies de la peau, du cuir chevelu et des muqueuses. Dans ce dernier cas, la composition topique comporte alors un principe actif qui peut par exemple consister en un agent anti-inflammatoire, un myorelaxant, un antifongique, un antibactérien ou antipelliculaire.

Lorsque la composition topique est utilisée en tant que composition cosmétique destinée à être appliquée sur la peau, sur le cuir chevelu ou les muqueuses, elle peut ou non comporter un principe actif, par exemple un agent hydratant, un agent bronzant, un filtre solaire, un antirides, un agent à visée amincissante, un agent antiradicalaire, un agent anti-acnéique, un antifongique ou antipelliculaire.

L'invention a enfin pour objet une composition topique selon l'invention comporte habituellement entre 0,1% et 10% massique et plus particulièrement de 1% à 5% massique du polyélectrolyte anionique tel que défini précédemment. Le pH de la composition topique est de préférence supérieur ou égal à 3.

La composition topique peut en outre comporter des composés classiquement compris dans ce type de compositions, par exemple des parfums, des conservateurs, des colorants, des pigments, des écrans solaires, des ingrédients actifs, des émollients ou des tensioactifs.

Selon un autre aspect particulier, l'invention concerne l'utilisation du polyélectrolyte anionique tel que défini précédemment, pour épaissir et émulsionner et stabiliser une composition topique comprenant au moins une phase aqueuse.

Le polyélectrolyte anionique selon l'invention est un substitut intéressant aux latex inverses vendus sous les noms de SEPIGEL™ 305, SEPIGEL™ 501, SIMULGEL™ EG, SIMULGEL™ EPG, SIMULGEL™ NS, SIMULGEL™ 600, SIMULGEL™ A, SEPIPLUS™ 265, SEPIPLUS™ 250, SEPIPLUS™ 400 ou SEPINOV™ EMT 10 par la demanderesse, car il présente aussi une bonne compatibilité avec les autres excipients utilisés pour la préparation de formulations telles que les laits, les lotions, les crèmes, les savons, les bains, les baumes, les shampooings ou les après-shampooings. Il peut aussi être mis en oeuvre avec lesdits SEPIGEL™ ou SIMULGEL™, SEPIPLUS™ et/ou SEPINOV™ EMT 10

Il est notamment compatible avec les concentrés décrits et revendiqués dans les publications internationales WO 92/06778, WO 95/04592, WO 95/13863, WO 96/37285, WO 98/22207, WO 98/47610 ou dans FR 2 734 496, avec les agents tensioactifs décrits dans WO 93/08204.Il est particulièrement compatible avec le MONTANOV™ 68, le MONTANOV™ 82, le MONTANOV™ 202, le MONTANOV™ L, le MONTANOV™ S, le FLUIDANOV™ 20X ou l'EASYNOV™. Il peut également être utilisé dans des émulsions du type de celles décrites et revendiquées dans EP 0 629 396 et dans les dispersions aqueuses cosmétiquement ou physiologiquement acceptable avec un composé organopolysiloxane choisi, par exemple parmi ceux décrits dans WO 93/05762 ou dans WO 93/21316.

Il peut également être utilisé pour former des gels aqueux à pH acide cosmétiquement ou physiologiquement acceptables, tels que ceux décrit dans WO 93/07856; elle peut également être utilisée en association avec des celluloses non-ioniques, pour former par exemple des gels de coiffage tels que ceux décrits dans EP 0 684 024, ou encore en association avec des esters d'acides gras et de sucre, pour former des compositions pour le traitement du cheveu ou de la peau telles que celles décrites dans EP 0 603 019, ou encore dans les shampooings ou après-shampooings tels que décrits et revendiqués dans WO 92/21316 ou enfin en association avec un homopolymère anionique tels que le CARBOPOL™ pour former des produits de traitement des cheveux comme ceux décrits dans DE 19523596.

Il est également compatible avec les dérivés N-acylés d'aminoacides, ce qui permet son utilisation dans des compositions apaisantes notamment pour peau sensible, telles que celles décrites ou revendiquées dans WO 92/21318, WO 94/27561 ou WO 98/09611. Il est également compatible avec des polymères épaississants et/ou gélifiants comme les hydrocolloïdes d'origine végétale ou biosynthétique, par exemple la gomme xanthane, la gomme de karaya, les carraghénates, les alginates, les galactomannanes ; comme les silicates ; comme la cellulose et ses dérivés ; comme l'amidon et ses dérivés hydrophiles ; comme les polyuréthanes.

### A) Préparation de polyélectrolytes :

### Exemple 1 (selon l'invention) : Terpolymère de 2-méthyl 2-[(1-oxo 2-propènyl) amino] 1-propanesulfonate d'ammonium, N,N-diméthyl acrylamide et méthacrylate de lauryle tétraéthoxylé [AMPS/DMAM/MAL(4OE) 77,4/19,2/3,4 molaire], réticulé au triméthylol propanetriacrylate (TMPTA).

On charge dans un réacteur maintenu à 25°C sous agitation, 592g d'une solution aqueuse à 15% massique de 2-méthyl 2-[(1-oxo 2-propènyl) amino] 1-propanesulfonate d'ammonium dans un mélange tert-butanol/eau (97,5/2,5 en volume), 10,1g de N,N-diméthyl acrylamide, 4,2g de méthacrylate de lauryle tétraéthoxylé et 0,75g de triméthylol propanetriacrylate. Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C. Après refroidissement, la poudre qui s'est formée en cours de polymérisation, est filtrée et séchée pour obtenir le produit désiré, dénommé par la suite : Polyélectrolyte 1.

### Exemple 2 (selon l'invention) : Terpolymère de 2-méthyl 2-[(1-oxo 2-propènyl) amino] 1-propanesulfonate d'ammonium, N,N-diméthyl acrylamide et méthacrylate de stéaryle d'eicosaéthoxylé [AMPS/DMAM/MAS(20OE) 79,3/19,7/1 molaire], réticulé au triméthylol propanetriacrylate (TMPTA).

On charge dans un réacteur maintenu à 25°C sous agitation, 592g d'une solution aqueuse à 15% massique de 2-méthyl 2-[(1-oxo 2-propènyl) amino] 1-propanesulfonate d'ammonium dans un mélange tert-butanol/eau (97,5/2,5 en volume), 10,1g de N,N-diméthyl acrylamide, 6,2g de méthacrylate de stéaryle d'eicosaéthoxylé et 0,75g de triméthylol propanetriacrylate. Après un temps suffisant pour atteindre une bonne homogénéisation de la solution, celle-ci est désoxygénée par barbotage d'azote

chauffée à 70°C. 0,42g de peroxyde de dilauroyle sont alors ajoutés et le milieu réactionnel est ensuite maintenu pendant environ 60 minutes à 70°C puis 2 heures à 80°C. Après refroidissement, la poudre qui s'est formée en cours de polymérisation, est filtrée et séchée pour obtenir le produit désiré, dénommé par la suite : Polyélectrolyte 2.

### Exemple T1 (selon l'état de la technique) : Terpolymère de 2-méthyl 2-[(1-oxo 2-propènyl) amino] 1-propanesulfonate d'ammonium, de N,N-diméthyl acrylamide et d'acrylate de lauryle tétraéthoxylé, [AMPS/DMAM/AL(4OE), 77,4/19,2/3,4] réticulé au triméthylol propanetriacrylate (TMPTA)

On charge dans un réacteur maintenu à 25°C, sous agitation 592 g d'une solution du sel d'ammoniaque de l'acide 2-méthyl 2-[(1-oxo 2-propènyl) amino] 1-propanesulfonique à 15,5% dans un mélange tert-butanol/eau (97,5/2,5 en volume) 10,1 g de N,N-diméthyl acrylamide, 4,1 g d'acrylate de lauryle tétraéthoxylé et 0,75 g de triméthylol propanetriacrylate.

Après un temps suffisant pour atteindre une bonne homogénéisation de la solution, on désoxygène par barbotage d'azote puis on porte la température du milieu à 70°C. Lorsque la température désirée est atteinte, 0,42 g de peroxyde de dilauroyle sont ajoutés et le milieu réactionnel est ensuite maintenu pendant environ 60 minutes à 70°C puis 2 heures à 80°C. Après refroidissement, la poudre qui s'est formée en cours de polymérisation, est filtrée et séchée pour obtenir le produit désiré, nommé par la suite : Polyélectrolyte T1.

### Exemple T2 (selon l'état de la technique) : Terpolymère de 2-méthyl 2-[(1-oxo 2-propènyl) amino] 1-propanesulfonate d'ammonium, de N,N-diméthyl acrylamide et d'acrylate de stéaryle d'eicosaéthoxylé [AMPS/DMAM/AS(20OE), 79,3/19,7/1 molaire] réticulé au triméthylol propanetriacrylate

On charge dans un réacteur maintenu à 25°C, sous agitation 592 g d'une solution du sel d'ammoniaque de l'acide 2-méthyl 2-[(1-oxo 2-propènyl) amino] 1-propanesulfonique à 15,5% dans un mélange tert-butanol / eau (97,5 / 2,5 en volume), 10,1 g de N,N-diméthyl acrylamide, 6,1 g de méthacrylate de stéaryle d'eicosaéthoxylé et 0,5 g de triméthylol propanetriacrylate. Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C. Après refroidissement, la poudre qui s'est formée en cours de polymérisation, est filtrée et séchée pour obtenir le produit désiré, dénommé par la suite : Polyélectrolyte T2

### Exemple T3 (selon l'état de la technique) : Terpolymère de 2-méthyl 2-[(1-oxo 2-propènyl) amino] 1-propanesulfonate d'ammonium, de N,N-diméthyl acrylamide et de méthacrylate de béhènyle pentacosaéthoxylé [AMPS/DMAM/MAB(25OE), 79,3/19,7/1], réticulé au triméthylol propanetriacrylate ;

On charge dans un réacteur maintenu à 25°C, sous agitation 592 g d'une solution du sel d'ammoniaque de l'acide 2-méthyl 2-[(1-oxo 2-propènyl) amino] 1-propanesulfonique à 15,5% dans un mélange tert-butanol/eau (97,5/2,5 en volume), 10,1 g de N,N-diméthyl acrylamide, 7,7 g de méthacrylate de béhènyle pentacosaéthoxylé, (1500 =PM) et 0,75 g de triméthylol propanetriacrylate, Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C. Après refroidissement, la poudre qui s'est formée en cours de polymérisation, est filtrée et séchée pour obtenir le produit désiré, dénommé par la suite : Polyélectrolyte T3.

### B) Etudes comparatives :

Les épaississants selon l'invention et ceux selon l'état de la technique ont été évalués comme suit :
**a) -** Mesure des viscosités (η) :
- à 2% massique dans l'eau,
- à 2% massique dans l'eau à pH 3,5
- et à 2% massique dans l'eau à pH 3,5 et comprenant 2% de chlorure de sodium.

Ces mesures de viscosité, exprimées en mPas, ont été effectuées à 25°C au moyen d'un rhéomètre Brookfield de type RVT équipé de l'axe numéro 6 et réglé à la vitesse de rotation de 5 tours par minute (rpm). Le pH a été réglé à 3,5 par ajout d'acide glycolique et si nécessaire de soude. Les mesures ont été effectuées juste après réalisation T = 0 à T = 1 mois (M1) puis à T = 3 mois (M3).
**b) -** On a préparé des formules applicatives d'évaluation épaissies avec chacun des polyélectrolytes 1, 2, 3, 4, T1, T2 et T3 de composition suivante :

| | |
|---|---|
| Acide glycolique : | 4% massique |
| Epaississant : | 2% massique |
| Isohexadécane : | 15% massique |
| Mélange d'alcool cétylique et alcool béhènylique : | 2% massique |
| SEPICIDE™ HB : | 0,5% massique |
| NaOH : | qs pH 3.5 |
| Eau : | qsp |

Ces formules sont évaluées en termes d'aspect visuel, de propriétés sensorielles et de stabilité de la formule après 24 heures. Les propriétés mises en évidence pour les polyélectrolytes et les compositions selon l'invention sont consignées dans le tableau suivant où elles sont mises en perspective avec des polyélectrolytes et composition s selon l'état de la techniques.

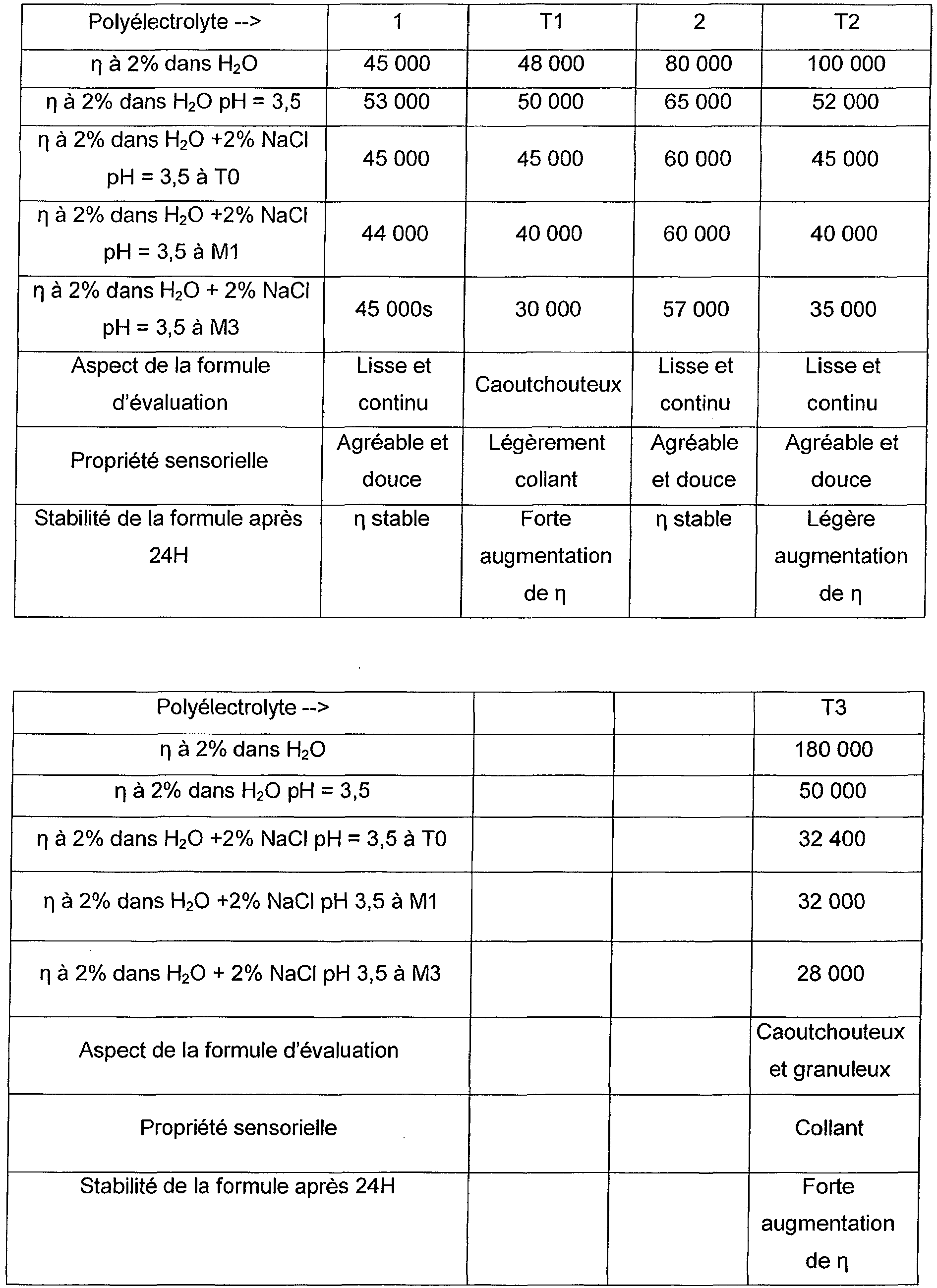

Ces résultats mettent en évidence que les polyélectrolytes objets de la présente invention, induisent des compositions plus stables aux sels, grâce à la présence en leur sein du monomère de formule (I).

### C) Exemples de formulations préparées avec les polyélectrolytes 1 ou 2 selon l'invention

### Exemple 3 : Crème de soin (proportions exprimées en pourcentages massique)

| | | |
|---|---|---|
| Cyclométhicone : | | 10% |
| Polyélectrolyte 1 : | | 0,8% |
| MONTANOV™ 68 : | | 2% |
| Alcool stéarylique : | | 1% |
| Alcool stéarique : | | 0,5% |
| Conservateur : | | 0,65% |
| Lysine : | | 0,025% |
| EDTA (sel disodique) : | | 0,05% |
| Gomme de xanthane : | | 0,2% |
| Glycérine : | | 3% |
| Eau : | q.s.p. | 100% |

### Exemple 4 : Baume après-rasage

### FORMULE

| | | | |
|---|---|---|---|
| A | Polyélectrolyte 2 : | | 1,5% |
| | Eau : | q.s.p. | 100% |
| B | MICROPEARL™ M 100 : | | 5,0% |
| | SEPICIDE™ CI : | | 0,50% |
| | Parfum : | | 0,20% |
| | Ethanol 95° : | | 10,0% |

### MODE OPERATOIRE

Ajouter B dans A.

### Exemple 5 : Gel de massage

### FORMULE

| | | |
|---|---|---|
| A | Polyélectrolyte 2 : | 3,5% |
| | Eau : | 20,0% |
| B | Colorant : | 2 gouttes/100g |
| | Eau : | q.s. |
| C | Ethanol : | 10% |
| | Menthol : | 0,10% |
| D | Huile de silicone : | 5,0% |

### MODE OPERATOIRE

Ajouter B dans A, puis ajouter au mélange, C puis D

### Exemple 5 : Fond de teint hydratant et matifiant

### FORMULE

| | | | |
|---|---|---|---|
| A | eau : | | 20,0% |
| | Butylène glycol : | | 4,0% |
| | PEG-400 : | | 4,0% |
| | PECOSIL™ PS100 : | | 1,0% |
| | Hydroxyde de Sodium : | q.s. | pH = 9 |
| | Dioxyde de titane : | | 7,0% |
| | Talc : | | 2,0% |
| | Oxyde de fer jaune : | | 0,8% |
| | Oxyde de fer rouge : | | 0,3% |
| | Oxyde de fer noir : | | 0,05% |
| B | LANOL™ 99 : | | 8% |
| | Caprylic capric triglycéride | | 8% |
| | MONTANOV™ 202 : | | 5,00% |
| C | eau : | q.s.p. | 100% |
| | MICROPEARL™ M305 : | | 2,0% |
| | EDTA tétrasodé : | | 0,05% |
| D | Cyclométhicone : | | 4,0% |
| | Gomme de Xanthane : | | 0,2% |
| | Polyélectrolyte 1 : | | 0,8% |
| E | SEPICIDE™ HB : | | 0,5% |
| | SEPICIDE CI : | | 03% |
| | Parfum : | | 0,2% |

### MODE OPERATOIRE

Préparer à 80°C, les mélanges B + D et A + C, puis mélanger et émulsionner l'ensemble.

### Exemple 6 : Gel coup d'éclat

### FORMULE

| | | | |
|---|---|---|---|
| A | Polyélectrolyte 1 : | | 4% |
| | Eau : | | 30% |
| B | ELASTINE HPM : | | 5,0% |
| C | MICROPEARL™ M 100 : | | 3% |
| | Eau : | | 5% |
| D | SEPICIDE™ CI : | | 0,2% |
| | SEPICIDE™ HB : | | 0,3% |
| | Parfum : | | 0,06% |
| | Pyrolidinonecarboxylate de sodium 50% : | | 1% |
| | Eau : | q. s. p. | 100% |

### MODE OPERATOIRE

Préparer A ; additionner B, puis C, puis D.

### Exemple 7 : Crème hydratante pour peaux grasses

| | | |
|---|---|---|
| MONTANOV™ 68 : | | 5% |
| Cétylstéaryloctanoate : | | 8% |
| Palmitate d'octyle : | | 2% |
| Eau : | q.s.p. | 100% |
| Polyélectrolyte 1 : | | 0,6% |
| MICROPEARL™ M100 : | | 3,0% |
| Mucopolysaccharides : | | 5% |
| SEPICIDE™ HB : | | 0,8% |
| Parfum: | | 0,3% |

### Exemple 8 : Baume après-rasage apaisant sans alcool

| | | |
|---|---|---|
| LIPACIDE™ PVB : | | 1,0% |
| LANOL™ 99: | | 2,0% |
| Huile d'amandes douces : | | 0,5% |
| Polyélectrolyte 1 : | | 3,5% |
| Eau : | q.s.p. | 100% |
| Parfum : | | 0,4% |
| SEPICIDE™ HB : | | 0,4% |
| SEPICIDE™ CI : | | 0,2% |

### Exemple 9 : Soin apaisant après soleil

| | | |
|---|---|---|
| Mélange de N-lauroyl aminoacides : | | 0,1% à 5% |
| Aspartate de magnésium et de potassium : | | 0,002% à 0,5% |
| LANOL™ 99: | | 10,0% |
| Eau : | q.s.p. | 100% |
| Polyélectrolyte 1 : | | 2,50% |
| SEPICIDE™ HB : | | 0,3% |
| SEPICIDE™ CI : | | 0,2% |
| Parfum : | | 0,4% |
| Colorant : | | 0,03% |

### Exemple 10 : Lait démaquillant

| | | |
|---|---|---|
| MONTANOV™ S : | | 3% |
| PRIMOL™ 352 : | | 8,0% |
| Huile d'amandes douces : | | 2% |
| Eau : | q.s.p. | 100% |
| Polyélectrolyte 2 : | | 0,8% |
| Conservateur : | | 0,2% |

### Exemple 11: Gel contour des yeux

| | | |
|---|---|---|
| Polyélectrolyte 2 : | | 2,0% |
| Parfum : | | 0,06% |
| Pyrolidinonecarboxylate de sodium : | | 0,2% |
| DOW CORNING™ 245 Fluid : | | 2,0% |
| Eau : | q. s. p. | 100% |

### Exemple 12 : Composition de soin non rincée

| | | |
|---|---|---|
| Polyélectrolyte 2 : | | 1,5% |
| Parfum : | | q. s |
| Conservateur : | | q. s. |
| DOW CORNING™ X2 8360 : | | 5,0% |
| DOW CORNING™ Q2 1401 : | | 15,0% |
| Eau : | q.s.p. | 100% |

### Exemple 13 : Gel crème teinté ultra naturel

### FORMULE

| | | | |
|---|---|---|---|
| A | Eau: | | 10,0% |
| | Butylène glycol : | | 4,0% |
| | PEG-400: | | 4,0% |
| | PECOSIL™ PS100 : | | 1,5% |
| | NaOH : | q.s. | pH = 7 |
| | Dioxyde de titane : | | 2,0% |
| | Oxyde de fer jaune : | | 0,8% |
| | Oxyde de fer rouge : | | 0,3% |
| | Oxyde de fer noir : | | 0,05% |
| B | LANOL™ 99: | | 4,0% |
| | Caprylic capric triglycéride | | 4,0% |
| | SEPIFEEL™ ONE : | | 1,0% |
| | Polyélectrolyte 2 : | | 3,0% |
| C | Eau : | q.s.p. | 100% |
| | MICROPEARL™ M305 : | | 2,0% |
| | EDTA tétrasodé : | | 0,05% |
| | Cyclométhicone : | | 4,0% |
| D | SEPICIDE™ HB : | | 0,5% |
| | SEPICIDE CI : | | 03% |
| | Parfum : | | 0,2% |

### MODE OPERATOIRE

Préparer le mélange B + C puis ajouter A puis D.

### Exemple 14 : Lait solaire au monoï de Tahiti

| | | |
|---|---|---|
| Monoï de Tahiti : | | 10% |
| LIPACIDE™ PVB : | | 0,5% |
| Polyélectrolyte 1 : | | 2,2% |
| Eau: | q.s.p. | 100% |
| Parfum : | | 0,1% |
| SEPICIDE™ HB : | | 0,3% |
| SEPICIDE™ CI: | | 0,1% |
| PARSOL™ MCX : | | 4,0% |

### Exemple 15 : Soin solaire pour le visage

| | | |
|---|---|---|
| Cyclométhicone et Diméthiconol : | | 4,0% |
| Polyélectrolyte 2 : | | 3,5% |
| Eau : | q.s.p. | 100% |
| Parfum: | | 0,1% |
| SEPICIDE™ HB : | | 0,3% |
| SEPICIDE™ CI : | | 0,21% |
| PARSOL™ MCX : | | 5,0% |
| Micatitane : | | 2,0% |
| Acide lactique : | q.s.p. | pH = 6,5 |

### Exemple 16 : Lait corporel

### FORMULE

| | | | |
|---|---|---|---|
| A | SIMULSOL™ 165: | | 5,0% |
| | LANOL™ 1688 : | | 12,0% |
| | LANOL™ 14 M : | | 2,0% |
| | Alcool cétylique : | | 0,3% |
| | SCHERCEMOL™ OP : | | 3% |
| B | Eau : | q.s.p. | 100% |
| C | Polyélectrolyte 2 : | | 0,35% |
| D | SEPICIDE™ CI : | | 0,2% |
| | SEPICIDE™ HB : | | 0,5% |
| | Parfum: | | 0,20% |

### MODE OPERATOIRE

Emulsionner B dans A vers 75°C ; ajouter C vers 60°C, puis D vers 30°C

### Exemple 17 : Gel soin de message

### FORMULE

| | | | |
|---|---|---|---|
| A | Polyélectrolyte 1 : | | 3,00% |
| | Eau : | | 30% |
| B | SEPICIDE™ CI : | | 0,20% |
| | SEPICIDE™ HB : | | 0,30% |
| | Parfum : | | 0,05% |
| C | Colorant : | | q.s. |
| | Eau : | q.s.p. | 100% |
| D | MICROPEARL™ SQL : | | 5,0% |
| | LANOL™ 1688 : | | 2% |

### MODE OPERATOIRE

Préparer A ; additionner B, puis C, puis D.

### Exemple 18 : Lait corporel

### FORMULE

| | | | |
|---|---|---|---|
| A | MONTANOV™ S : | | 3,0% |
| | Triheptonate de glycérol : | | 10,0% |
| B | Eau : | q.s.p. | 100% |
| C | Polyélectrolyte 1 : | | 1,0% |
| D | Parfum : | | q.s. |
| | Conservateur : | | q.s. |

### MODE OPERATOIRE

Fondre A à environ 75°C. Emulsionner B dans A à 75°C puis ajouter C vers 60°C, puis D.

### Exemple 19 : Baume après-rasage apaisant sans alcool

| | | |
|---|---|---|
| LIPACIDE™ PVB : | | 1,0% |
| LANOL™ 99 : | | 2,0% |
| Huile d'amandes douces : | | 0,5% |
| Polyélectrolyte 1 : | | 3,5% |
| Eau : | q.s.p. | 100% |
| Parfum : | | 0,4% |
| SEPICIDE™ HB : | | 0,4% |
| SEPICIDE™ CI : | | 0,2% |

### Exemple 20 : Gel rafraîchissant après-rasage

| | | |
|---|---|---|
| LIPACIDE™ PVB : | | 0,5% |
| LANOL™ 99 : | | 5,0% |
| Polyélectrolyte 1 : | | 2,5% |
| Eau: | q.s.p. | 100% |
| MICROPEARL™ LM : | | 0,5% |
| Parfum : | | 0,2% |
| SEPICIDE™ HB : | | 0,3% |
| SEPICIDE™ CI: | | 0,2% |

### Exemple 21 : Gel brillance

| | | |
|---|---|---|
| Polyélectrolyte 1 : | | 1,5% |
| Silicone volatile : | | 25% |
| Monopropylèneglycol : | | 25% |
| Eau déminéralisée : | | 10% |
| Glycérine: | qsp | 100% |

### Exemple 22 : Gel amincissant

| | | |
|---|---|---|
| Polyélectrolyte 2 : | | 1,5% |
| Isononanoate d'isononyle : | | 2% |
| Caféine : | | 5% |
| Ethanol : | | 40% |
| MICROPEARL™ LM : | | 2% |
| Eau déminéralisée : | qsp | 100% |
| Conservateur parfum : | | qs |

### Exemple 23 : Gel de soin peaux mixtes

| | | |
|---|---|---|
| Polyélectrolyte 2 : | | 4% |
| Squalane végétal : | | 5% |
| Dimethicone : | | 1,5% |
| SEPICONTROL™ A5 : | | 4% |
| Gomme xanthane : | | 0,3% |
| Eau : | qsp : | 100% |
| Conservateur, Parfum : | | qs. |

### Exemple 24 : Lotion capillaire

| | | |
|---|---|---|
| Butylène glycol : | | 3,0% |
| Polyélectrolyte 2 : | | 3% |
| SIMULSOL™1293 : | | 3,0% |
| Acide lactique : | qs. pH | = 6 |
| SEPICIDE™ HB : | | 0,2% |
| SEPICIDE™CI : | | 0,3% |
| Parfum : | | 0,3% |
| Eau : | | qs. 100% |

### Exemple 25 : Protecteur "leave-on" ; Soin anti-stress pour cheveux

| | | |
|---|---|---|
| KETROL™T : | | 0,5% |
| Mélange de cocoyl aminoacides : | | 3,0% |
| Butylèneglycol : | | 5,0% |
| DC 1501 : | | 5,0% |
| Polyélectrolyte 2 : | | 4,0% |
| SEPICIDE™ HB: | | 0,5% |
| SEPICIDE™CI : | | 0,3% |
| Parfum : | | 0,3% |
| Eau : | qsp. | 100 |

### Exemple 26 : Poudre pressée Terre de soleil

| | |
|---|---|
| Polyélectrolyte 1 : | 2,00% |
| Lanol™ 99 : | 12,00% |
| SEPIWHITE™ MSH : | 1,00% |
| Talc : | 33,00% |
| MICROPEARL™ M310 : | 3,00% |
| Oxyde de fer jaune : | 0,80% |
| Oxyde de fer rouge : | 0,30% |
| Oxyde de fer noir : | 0,05% |
| Mica : | qs 100% |

### Exemple 27 : Emulsion pour peaux à tendance atopique

| | |
|---|---|
| ARLACEL™ P135 : | 2,00% |
| Polyélectrolyte 2 : | 1,00% |
| Lanol™ 1688 : | 14,00% |
| Primol™ 352 : | 8,00% |
| Glycérine : | 5,00% |
| Eau : | qsp 100% |
| Sulfate de magnésium : | 0,70% |
| SEPICIDE™ HB : | 0,30% |
| SEPICIDE™ CI : | 0,20% |
| MICROPEARL™ M310 : | 5,00% |

### Exemple 28 : Gel solaire et autobronzant

| | | |
|---|---|---|
| MONTANOV™ S : | | 3,0% |
| Triheptanoate de glycéryle : | | 10,0% |
| LIPACIDE™ PVB : | | 1,05% |
| Polyélectrolyte 2 : | | 2,2% |
| Eau : | qs. | 100% |
| Dihydroxyacétone : | | 5% |
| Parfum : | | 0,1% |
| SEPICIDE™ HB : | | 0,3% |
| SEPICIDE™ CI: | | 0,1% |
| PARSOL™ MCX : | | 4,0% |

### Exemple 29 : Crème autobronzante aux α-hydroxy acides

| | | |
|---|---|---|
| MONTANOV™ 68 : | | 5,0% |
| LIPACIDE™ PVB : | | 1,05% |
| Lanol™ 99 : | | 10,0% |
| Eau: | qs. | 100% |
| Acide gluconique : | | 1,5% |
| Dihydroxyacétone : | | 3% |
| Triéthanolamine : | | 0,9% |
| Polyélectrolyte 1 : | | 1,5% |
| Parfum : | | 0,4% |
| SEPICIDE™ HB : | | 0,2% |
| SEPICIDE™ CI : | | 0,4% |

### Exemple 30 : Crème autobronzante aux α-hydroxy acides pour peau sensible

| | | |
|---|---|---|
| Mélange de N-lauroyl aminoacides : | | 0,1% à 5% |
| Aspartate de magnésium et de potassium : | | 0,002% à 0,5% |
| MONTANOV™ 68 : | | 5,0% |
| Lanol™ 99 : | | 2,0% |
| Eau : | qs. | 100% |
| Acide lactique : | | 1,5% |
| Dihydroxyacétone : | | 3,5% |
| Triéthanolamine : | | 0,9% |
| Polyélectrolyte 1 : | | 1,5% |
| Parfum : | | 0,4% |
| SEPICIDE™ HB : | | 0,3% |
| SEPICIDE™ CI : | | 0,2% |

Les définitions des produits commerciaux utilisés dans les exemples, sont les suivantes :
SIMULSOL™ 1293 est de l'huile de castor hydrogénée et éthoxylée, avec un indice d'éthoxylation égal à 40, commercialisé par la société SEPPIC.
KETROL™ T est de la gomme de xanthane commercialisée par la société KELCO.
LANOL™ 99 est de l'isononanoate d'isononyle commercialisé par la société SEPPIC.
DC1501 est un mélange de cyclopentasiloxane et de diméthiconol commercialisé par la société DOW CHEMICAL.
Le MONTANOV™ 68 (cétéaryl glucoside), est une composition auto-émulsionnable telle que décrite dans WO 92/06778, commercialisée par la société SEPPIC.
Le MICROPEARL™ M 100 est une poudre ultra fine au toucher très doux et à action matifiante commercialisée par la société MATSUMO
Le SEPICIDE™ CI, imidazolidine urée, est un agent conservateur commercialisé par la société SEPPIC.
Le SIMULSOL™ 165 est du stéarate de glycérol auto-émulsionnable commercialisée par la société SEPPIC.
Le LANOL™ 1688 est un ester émollient à effet non gras commercialisé par la société SEPPIC.
Le LANOL™ 14M est un facteur de consistance commercialisés par la société SEPPIC.
Le SEPICIDE™ HB, qui est un mélange de phénoxyéthanol, de méthylparaben, d'éthylparaben, de propylparaben et de butylparaben, est un agent conservateur commercialisé par la société SEPPIC.
Le SCHERCEMOL™ OP est un ester émollient à effet non gras.
Le PARSOL™ MCX est du para-méthoxy cinnamate d'octyle ; commercialisé par la société GIVAUDAN.
Le MONTANOV™ S est un agent nacrant, commercialisé par la société SEPPIC, à base d'un mélange d'alkyl poly glucosides tels que ceux décrits dans WO 95/13863.
Le MICROPEARL™ SQL est un mélange de micro particules renfermant du squalane qui se libère sous l'action du massage; il est commercialisé par la société MATSUMO.
Le DOW CORNING™ 245 Fluid est de la cyclométhicone, commercialisée par la société DOW CORNING.
Le LIPACIDE™ PVB, est un hydrolysât de protéines de blé acylé commercialisé par la société SEPPIC.
Le MICROPEARL™ LM est un mélange de squalane, de polyméthylméthacrylate et de menthol, commercialisé par la société SEPPIC.
Le SEPICONTROL™ A5 est un mélange capryloy glycine, sarcosine, extrait de cinnamon zylanicum, commercialisé par la société SEPPIC, tel que ceux décrits dans la demande internationale de brevet PCT/FR98/01313 déposée le 23 juin 1998.
Le MONTANOV™ 202, est une composition APG/alcools gras telle que décrite dans WO9 98/47610, commercialisée par la société SEPPIC.
Le SEPIWHITE™MSH est un actif dépigmentant (N-undecylenoyl phenylalanine) commercialisé par la société SEPPIC.
Le Z COTE HP1 est un oxyde de zinc micronisé ayant subi un traitement de surface distribué par GATTEFOSSE.
Le MICROPEARL™ M 310 est une poudre ultra fine au toucher très doux et à action matifiante commercialisée par la société MATSUMO.
Le PRIMOL™ 352 est une huile minérale commercialisée par la société EXXON.
Le PECOSIL™PS 100 est du Dimethicone PEG-7 commercialisé par la société PHOENIX.

## Revendications

1. Polyélectrolyte anionique réticulé, issu de la polymérisation de l'acide 2-méthyl 2-[(1-oxo 2-propènyl) amino] 1-propanesulfonique partiellement salifié ou totalement salifié, avec le N,N-diméthyl acrylamide, et au moins un monomère de formule (I) : 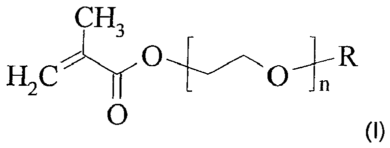 dans laquelle R représente un radical alkyle linéaire ou ramifié comportant de huit à vingt atomes de carbone et n représente un nombre supérieur ou égal à un et inférieur ou égal à vingt, choisi parmi le méthacrylate de lauryle tétraéthoxylé ou le méthacrylate de stéaryle eicosaéthoxylé, en présence d'au moins un agent de réticulation.

2. Polyélectrolyte anionique tel que défini à la revendication 1, pour lequel l'acide 2-méthyl 2-[(1-oxo 2-propènyl) amino] 1-propanesulfonique est partiellement salifié ou totalement salifié sous forme de sel de sodium, de sel de potassium ou de sel d'ammonium.

3. Polyélectrolyte anionique tel que défini à la revendication 2, pour lequel l'acide 2-méthyl 2-[(1-oxo 2-propènyl) amino] 1-propanesulfonique est partiellement ou totalement salifié sous forme de sel d'ammonium.

4. Polyélectrolyte anionique tel que défini à l'une quelconque des revendications 1 à 3, choisi parmi les polymères suivants :
- Terpolymère de l'acide 2-méthyl 2-[(1-oxo 2-propènyl) amino] 1-propanesulfonique totalement salifié sous forme de d'ammonium, du N,N-diméthyl acrylamide et du méthacrylate de lauryle tétraéthoxylé, réticulé au triméthylol propanetriacrylate ;
- Terpolymère de l'acide 2-méthyl 2-[(1-oxo 2-propènyl) amino] 1-propanesulfonique totalement salifié sous forme de sel d'ammonium, du N,N-diméthyl acrylamide et du méthacrylate de stéaryle d'eicosaéthoxylé, réticulé au triméthylol propanetriacrylate.

5. Polyélectrolyte anionique branché ou réticulé, tel que défini à l'une quelconque des revendications 1 à 4, comportant pour 100% de monomères mis en oeuvre :
- De 20% molaire à 80% molaire d'unités monomériques issues d'un monomère comportant une fonction acide fort ;
- De 15% molaire à 75% molaire d'unités monomériques issues d'un monomère neutre ;
- De 0,5% à 5% molaire d'unités monomériques issues d'un monomère de formule (I) telle que définie précédemment.

6. Polyélectrolyte anionique branché ou réticulé, tel que défini à la revendication 5, comportant pour 100% de monomères mis en oeuvre :
- De 55% molaire à 80% molaire d'unités monomériques issues d'un monomère comportant une fonction acide fort ;
- De 15% molaire à 40% molaire d'unités monomériques issues d'un monomère neutre ;
- De 1% à 5% molaire d'unités monomériques issues d'un monomère de formule (I) telle que définie précédemment.

7. Utilisation du polyélectrolyte anionique tel que défini à l'une quelconque des revendications 1 à 6, comme épaississant et/ou comme stabilisant et/ou comme émulsionnant, d'une composition topique cosmétique, dermopharmaceutique ou pharmaceutique.

8. Composition topique, comprenant de 0,1% et 10% massique du polyélectrolyte anionique tel que défini à l'une quelconque des revendications 1 à 6.

## Patentansprüche

1. Vernetzter anionischer Polymerelektrolyt, hervorgegangen aus der Polymerisation von 2-Methyl-2-[(1-oxo-2-propenyl)-amino]-1-propansulfonsäure, die teilweise oder vollständig in ein Salz überführt ist, mit N,N-Dimethylacrylamid, und mindestens einem Monomer der Formel (I): 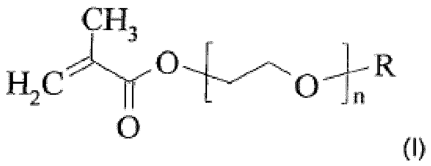 worin R ein geradkettiges oder verzweigtes Alkylradikal mit acht bis zwanzig Kohlenstoffatomen darstellt und n eine Zahl ist, die größer oder gleich eins und kleiner oder gleich zwanzig ist, ausgewählt aus tetraethoxyliertem Laurylmethacrylat oder eicosaethoxyliertem Stearylmethacrylat, in Gegenwart mindestens eines Vernetzungsmittels.

2. Anionischer Polymerelektrolyt nach Anspruch 1, wobei die 2-Methyl-2-[(1-oxo-2-propenyl)-amino]-1-propansulfonsäure teilweise oder vollständig in ein Salz überführt ist in Form von Natriumsalz, Kaliumsalz oder Ammoniumsalz.

3. Anionischer Polymerelektrolyt nach Anspruch 2, wobei die 2-Methyl-2-[(1-oxo-2-propenyl)-amino]-1-propansulfonsäure teilweise oder vollständig in ein Salz überführt ist in Form von Ammoniumsalz.

4. Anionischer Polymerelektrolyt nach einem der Ansprüche 1 bis 3, der ausgewählt ist aus den folgenden Polymeren:
- Terpolymer von 2-Methyl-2-[(1-oxo-2-propenyl)-amino]-1-propansulfonsäure, die vollständig in ein Salz überführt ist in Form von Ammoniumsalz, von N,N-Dimethylacrylamid und von tetraethoxyliertem Laurylmethacrylat, das mit Trimethylolpropantriacrylat vernetzt ist,
- Terpolymer von 2-Methyl-2-[(1-oxo-2-propenyl)-amino]-1-propansulfonsäure, die vollständig in ein Salz überführt ist in Form von Ammoniumsalz, von N,N-Dimethylacrylamid und von eicosaethoxyliertem Stearylmethacrylat, das mit Trimethylolpropantriacrylat vernetzt ist.

5. Verzweigter oder vernetzter anionischer Polymerelektrolyt nach einem der Ansprüche 1 bis 4, der für 100 % verwendeter Monomere Folgendes aufweist:
- 20 Mol-% bis 80 Mol-% Monomereinheiten, die aus einem Monomer hervorgegangen sind, das eine starke Säurefunktion aufweist;
15 Mol-% bis 75 Mol-% Monomereinheiten, die aus einem neutralen Monomer hervorgegangen sind;
- 0,5 Mol-% bis 5 Mol-% Monomereinheiten, die aus einem Monomer der Formel (I) hervorgegangen sind, wie diese oben definiert ist.

6. Verzweigter oder vernetzter anionischer Polymerelektrolyt nach Anspruch 5, der für 100 % verwendeter Monomere Folgendes aufweist:
- 55 Mol-% bis 80 Mol-% Monomereinheiten, die aus einem Monomer hervorgegangen sind, das eine starke Säurefunktion aufweist;
- 15 Mol-% bis 40 Mol-% Monomereinheiten, die aus einem neutralen Monomer hervorgegangen sind;
- 1 Mol-% bis 5 Mol-% Monomereinheiten, die aus einem Monomer der Formel (I) hervorgegangen sind, wie diese oben definiert ist.

7. Verwendung eines anionischen Polymerelektrolyten nach einem der Ansprüche 1 bis 6 als Verdickungsmittel und/oder Stabilisator und/oder Emulgator einer topischen, kosmetischen, dermopharmazeutischen oder pharmazeutischen Zusammensetzung.

8. Topische Zusammensetzung, die 0,1 Masse-% und 10 Masse-% des anionischen Polymerelektrolyten nach einem der Ansprüche 1 bis 6 enthält.

## Claims

1. Cross-linked anionic polyelectrolyte, resulting from the polymerisation of partially salified or completely salified 2-methyl 2-[(1-oxo 2-propenyl)amino] 1-propanesulfonic acid with N,N-dimethyl acrylamide and at least one monomer of formula (I): 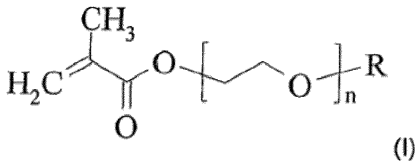 in which R represents a linear or branched alkyl radical having from eight to twenty carbon atoms and n represents a number greater than or equal to one and less than or equal to twenty, selected from tetraethoxylated lauryl methacrylate or eicosaethoxylated stearyl methacrylate, in the presence of at least one cross-linking agent.

2. Anionic polyelectrolyte according to claim 1, for which the 2-methyl 2-[(1-oxo 2-propenyl) amino] 1-propanesulfonic acid is partially salified or completely salified in the form of sodium salt, potassium salt or ammonium salt.

3. Anionic polyelectrolyte according to claim 2, for which the 2-methyl 2-[(1-oxo 2-propenyl) amino] 1-propanesulfonic acid is partially or completely salified in the form of ammonium salt.

4. Anionic polyelectrolyte according to any of claims 1 to 3, selected from the following polymers:
- terpolymer of 2-methyl 2-[(1-oxo 2-propenyl) amino] 1-propanesulfonic acid completely salified in the form of ammonium salt, N,N-dimethyl acrylamide and tetraethoxylated lauryl methacrylate, cross-linked with trimethylolpropane triacrylate;
- terpolymer of 2-methyl 2-[(1-oxo 2-propenyl) amino] 1-propanesulfonic acid completely salified in the form of ammonium salt, N,N-dimethyl acrylamide and eicosaethoxylated stearyl methacrylate, cross-linked with trimethylolpropane triacrylate.

5. Branched or cross-linked anionic polyelectrolyte according to any of claims 1 to 4, comprising for 100 % of monomers used:
- from 20 mol.% to 80 mol.% of monomer units derived from a monomer having a strong acid function;
- from 15 mol.% to 75 mol.% of monomer units derived from a neutral monomer;
- from 0.5 % to 5 mol.% of monomer units derived from a monomer of formula (I) as defined above.

6. Branched or cross-linked anionic polyelectrolyte according to claim 5, comprising for 100 % of monomers used:
- from 55 mol.% to 80 mol.% of monomer units derived from a monomer having a strong acid function;
- from 15 mol.% to 40 mol.% of monomer units derived from a neutral monomer;
- from 1 % to 5 mol.% of monomer units derived from a monomer of formula (I) as defined above.

7. Use of the anionic polyelectrolyte according to any of claims 1 to 6 as a thickener and/or as a stabiliser and/or as an emulsifier for a cosmetic, dermopharmaceutical or pharmaceutical topical composition.

8. Topical composition comprising between 0.1 and 10 wt.% of the anionic polyelectrolyte according to any of claims 1 to 6.
